Europäisches Patentamt

European Patent Office

Office européen des brevets

(19)

(11) Publication number: **0 072 232
B1**

(12) **EUROPEAN PATENT SPECIFICATION**

(45) Date of publication of patent specification: **30.07.86**

(21) Application number: **82304183.5**

(22) Date of filing: **09.08.82**

(51) Int. Cl.⁴: **A 61 B 17/12,** A 61 B 17/08, B 29 C 71/02

(54) Polyolefin surgical devices and method for producing the same.

(30) Priority: **10.08.81.US 291660**

(43) Date of publication of application:
**16.02.83 Bulletin 83/07**

(45) Publication of the grant of the patent:
**30.07.86 Bulletin 86/31**

(84) Designated Contracting States:
**DE FR GB IT SE**

(56) References cited:
**DE-B-1 229 278**
**GB-A-2 054 026**
**US-A-3 383 375**
**US-A-4 233 022**

**ENCYCLOPEDIA OF POLYMER SCIENCE AND
TECHNOLOGY, vol. 11, 1969, pages 608-609,
Interscience, New York, USA "Propylene
polymers"**

(73) Proprietor: **ETHICON INC.**
**U.S. Route 22**
**Somerville New Jersey 08876 (US)**

(72) Inventor: **Mellon, Darline A.**
**596 Country Club Road**
**Bridgewater New Jersey (US)**

(74) Representative: **Jones, Alan John et al**
**CARPMAELS & RANSFORD 43 Bloomsbury
Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The present invention relates to polyolefin surgical devices and more particularly to thermally formed polyolefin surgical devices having improved audible and tactile indications of functioning, hereinafter called audible and tactile response and improved functional integrity.

### Background of the invention

It is well known that in many and various surgical procedures, synthetic devices; that is, devices made from non-biological materials, are often implanted. Examples of such techniques are in surgery wherein various stainless steel or other metal clips are used to control bleeding in that they are used to ligate various blood vessels during the surgical procedure. Furthermore, in other surgical procedures, staples, metal rods, clips or sheets of material are implanted for various supports or other reasons in the surgical procedure. In most instances, these devices remain in the patient for considerable periods of time, though in some instances, they may be removed at some later date or even rejected by the natural physiological function of the human body.

Even though these metal surgical devices cause no harm from the medical viewpoint, it is often desired they not be allowed to remain in the body as they greatly disrupt the ability to subsequently use many of the new diagnostic treatments on the patient. The metal surgical devices disrupt X-ray imaging, computerized axial tomography imaging, and other of the new types of diagnostic imaging procedures. Hence, it is desirable that these surgical devices be replaced by plastic materials which do not have this disruptive effect on X-ray imaging or other types of diagnostic imaging procedures. However, in trying to develop the plastic materials to replace the metal materials, it has been found that it is very difficult to provide a combination of strength, flexibility, and functional characteristics in these plastic materials, to a degree that compares to the metal materials so that the plastic materials have not been readily accepted as substitutes for the metal materials. This is especially true in the smaller devices such as ligating clips and other types of clips used to ligate vessels or connect material such as tissue where the clip is small and the fine sizes of areas of the device require considerable strength, flexibility and functional integrity.

Also, metal devices such as ligating clips have marginal tactile properties to assist the doctor in the placement of the clips into areas where it is difficult to see. The prior art plastic devices are, for the most part, deficient in the desired level of tactile response as well as audible response to closure.

A class of material that has gained success in various types of surgical devices are the polyolefins. Polyolefin surgical devices have had considerable success because of their inertness and good strength. However, one of the problems with the polyolefin devices is their lack of audible and tactile responses. This is especially a problem when it is desired to use such devices for ligating clips where the surgeon may be using the clip in areas of limited vision and access.

### Summary of the invention

A new and improved, thermally formed, polyolefin sterile surgical device which has improved tactile response and improved audible response has been discovered.

Furthermore, the polyolefin sterile surgical devices of the present invention have good functional integrity; that is, the clip has good flexing characteristics yet it remains in position where placed.

In accordance with the present invention, the improved thermally formed, polyolefin devices have a crystallinity of at least 45% and preferably a crystallinity in excess of 50%. My preferred polyolefin surgical devices are made from polypropylene and are in the form of ligating clips; that is, clips comprising two leg members connected at their proximal ends by a resilient hinge means and containing some type of locking means to close the distal ends of the leg members. Such devices are disclosed in GB—A—2054026. In accordance with the present invention, the improved surgical devices are produced by heating the thermally formed device at a temperature of from 90°C to 150°C and preferably from between 100°C and 115°C, for a time sufficient to increase the crystallinity of the polyolefin to at least 45% and preferably to about 50%. It has been found that a time from about 20 minutes to three to four hours is satisfactory. In a preferred method of the present invention, an injection molded polypropylene ligating clip is heated at about 100°C for about 1 hour to provide the desired functional integrity and the audible and tactile responses in the ligating clip.

### Description of the drawings

Figure 1 is an enlarged perspective view of a ligating clip of the present invention;

Figure 2 is an enlarged perspective view of the clip of Figure 1 in position closing off a blood vessel;

Figure 3 is an enlarged perspective view of another surgical device of the present invention;

Figure 4 is a perspective view of a wound closure device of the present invention; and

Figure 5 is a cross-sectional view showing the device of Figure 4 in position to close a wound.

### Detailed description of the embodiments of the present invention

Though the present invention is applicable to many types of surgical devices thermally formed from polyolefin polymers, for the sake of clarity, it will be described in detail in conjunction with what are known as ligating clips made from the polyolefin polymers.

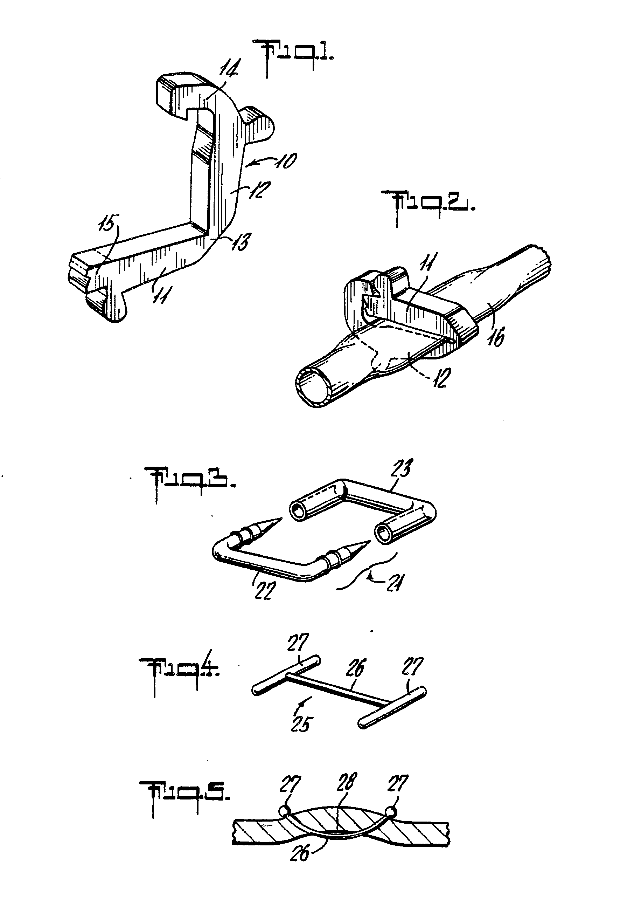

Referring to Figure 1, there is shown a ligating clip 10 of the present invention. The clip comprises a pair of leg members 11 and 12 which are attached at their proximal ends at the hinge area 13. The distal ends 14 and 15 of the leg members are configured in a manner so that they can be locked together when the clip is applied about a blood vessel 16 to close off the vessel as is more clearly seen in Figure 2. The hinge area, which is considerably thinner than the remainder of the clip, should be flexible yet strong. The body of the leg members should be strong and relatively rigid, and depending on the configuration of the distal ends, they should have a certain degree of flexibility. All in all, the clip in its entirety should have good functional integrity in that the hinge and locking areas should have desired flexibility but once the clip is closed and locked it should remain in that position.

Very often when the surgeon is using the ligating clip in a surgical procedure, he is working in an area where he has difficulty seeing or may not be able to see at all, those vessels he desires to ligate. Hence, he prefers that the hinge of the clip and the latching means both have some resistance to closing so that he can feel the clip closing as he applies pressure to the clip. The resistance to closing in the latch means; i.e., the tactile response may actually be the release or absence of resistance that is felt when the latch is fully engaged. This is termed the tactile response of the clip. Furthermore, the surgeon desires to know when the clip is fully closed. In the configuration shown in Figure 1 when the distal end of leg member 11 deflects to accept the distal end of leg member 12 and then snaps back to lock the distal end of leg member 12 in place, there is an audible 'click'. This is termed the audible response of the clip.

The clips according to the present invention are preferably made by the standard injection molding techniques well known for the molding of polyolefins. The parameters for the injection molding of polyolefins are well known to those skilled in the art and form no part of the present invention. The clips, once molded, are heat treated by heating the clips to temperatures of from 90°C, to approximately 150°C for periods of time from 20 minutes to 3 to 4 hours. The clips are treated under an inert atmosphere such as a dry nitrogen atmosphere to avoid oxidation and degradation of the clips at these temperatures. The resulting clips have good audible and tactile characteristics.

The polyolefin clips of the present invention are sterile and may be sterilized by the various procedures well known in the art for sterilizing polyolefin materials. A preferred method for sterilizing polyolefin materials is gas sterilization using ethylene oxide.

The following tests are used to determine the properties of surgical clips.

Opening strength
The opening strength of the clip is the force required to unlock the clip once it is locked. The strength is measured by closing a clip over two aligned strips of Mylar polyester film. The strips are 4 mm×178 mm×.076 mm thick. The strips are separated at the ends and each strip bent into a U configuration with the clip at the apex of the U. The open ends of the strips are clamped in the opposing jaws of an Instron Tensiometer using steel faced jaws. Using a strain rate of 5 mm/min the jaws are moved apart and the force necessary to open the clip is determined in kilograms.

Hinge strength
The hinge strength of the clip is the force required to break the clip at the hinge area. The strength is measured by cutting away the latching mechanism at the distal end of the clip. The cut ends of the clip are placed in the opposing jaws of an Instron Tensiometer. The jaws are steel faced. Using a strain rate of 5 mm/min the jaws are moved apart and the force necessary to break the hinge determined in kilograms.

Percent extension
The percent extension of the clip is a measure of the functional integrity and in part the dimensional stability of the clip. The percent extension of the clip is equivalent to apparent elongation of the clip determined from the Instron measurements and is calculated by the expression:

$$\% \ Extension = \frac{Velocity \ Crosshead \times Length \ of \ Chart}{Velocity \ Chart \times Gauge \ Length} \times 100$$

Crystallinity
The crystallinity of the clip is a measurement of the strength and functional integrity of the clip. X-ray diffraction is a convenient method of determining the amount and type of crystallinity in the clip. The X-ray crystallinity data is obtained using a Phillips vertical goniometer equipped with a graphite crystal monochrometer and scintillation detector interfaced to a strip chart recorder. CuKα radiation is employed and a sample is mounted and run using parafocusing geometry. The patterns obtained for a sample are analyzed for crystallinity and amorphous content using a duPont Curve Resolver.

The invention will be more fully described in conjunction with the following example.

Example 1
Ligating clips having the configuration as depicted in Figure 1 are molded in a multi cavity mold in an injection molding machine utilizing a polypropylene resin as described in U.S. Patent 3,630,205. The clips are molded at a nozzle or melt temperature of about 180°C and a mold temperature of 40°C. The clips as molded are tested for opening strength, hinge strength, and crystallinity. The opening strength, hinge strength and crystallinity are determined as previously described. The clips as injection molded, have an opening strength of 2.18 kilograms, a hinge strength of 2.25 kilograms, and a crystallinity of 41%. The clips when closed about a thin piece of film, have no tactile response and virtually no audible response. The clips as molded are heated for a period of one hour at a temperature of 100°C in a forced air oven. The opening strength of the heat-treated clip is 2.1 kilograms and the hinge strength is 2.8 kilograms. The crystallinity has been increased to 54% and the clips have good tactile response and good audible response.

Example 2

Ligating clips are molded as described in Example 1. The clips as molded are tested for opening strength, hinge strength, and percent extension. The opening strength of the molded clip is 2.20 kilograms, the hinge strength is 3.24 kilograms, and the percent extension is 233 percent. Some of the molded clips are heated at various temperatures and times as outlined in the following table. The heating is accomplished in a dry nitrogen atmosphere. The nitrogen is 99.95% pure, contains a maximum of 1 ppm oxygen and a maximum of 3.6 ppm water. The treated clips are tested for opening strength, hinge strength and percent extension. The results are given in the following table.

TABLE 1

| Temperature (°C) | Cycle (hrs) | Opening strength (kg) | Hinge strength (kg) | Extension at break (%) | Crystal-linity (%) |
|---|---|---|---|---|---|
| As molded | — | 2.20 | 3.24 | 233 | 43 |
| 90 | 2 | 2.26 | 3.46 | 100 | 50 |
| | 4 | 2.25 | 3.47 | 100 | 52 |
| 100 | 0.5 | 2.17 | 3.66 | 88 | 48 |
| | 1 | 2.00 | 3.62 | 86 | 48 |
| | 2 | 2.14 | 3.66 | 83 | 51 |
| 110 | 0.5 | 2.09 | 3.63 | 76 | 51 |
| | 1 | 2.17 | 3.63 | 69 | 50 |
| | 2 | 2.05 | 3.73 | 78 | 49 |
| 115 | 0.5 | 2.09 | 3.77 | 79 | 52 |
| | 1 | 1.91 | 3.76 | 76 | 55 |
| | 2 | 1.90 | 3.80 | 69 | 53 |
| 130 | 0.3 | 2.15 | 3.92 | 54 | 60 |
| 140 | 0.3 | 2.13 | 4.06 | 61 | 57 |
| 150 | 0.3 | 2.10 | 4.12 | 55 | 68 |

Though I have described the present invention in conjunction with ligating clips, it should be apparent that other types of surgical devices may also benefit from the present invention. For example, in Figure 3, there is shown a 2-piece fastening device 21 in accordance with the present invention. The device includes a staple member 22 and an engaging member 23 and may be used to fasten together fascia or portions of internal organs and the like.

In Figure 4, there is shown yet another surgical device 25 for closing a wound. This device comprises a thin rod like member 26 with a bar 27 attached at each end. The device is used to close a wound in the manner depicted in Figure 5. The bars 27 lie on either side of the wound 28 and the wound itself is spanned by the connecting rod 26.

Other molded products contemplated by the present invention are clamps, clips, staples, buttons, snaps, surgical instruments, and the like.

The molded devices of the present invention may be made from any of the polyolefins; that is, polyethylene, polypropylene and the like. A specifically suitable polypropylene for use in molding the surgical devices of the present invention is described in U.S. Patent 3,630,205 issued December 28, 1971.

## Claims

1. A thermally formed polyolefin surgical device having both audible and tactile indications of functioning and functional integrity, said polyolefin have a crystallinity in excess of 45%.

2. An injection molded surgical device according to Claim 1 wherein the polyolefin is polypropylene.

3. A surgical device according to Claim 1 or Claim 2 wherein the surgical device is a ligating clip.

4. A surgical device according to Claims 1, 2, or 3 wherein the crystallinity is in excess of 50%.

5. An injection molded polypropylene ligating clip having two leg members connected at their proximal ends by a resilient hinge portion and containing locking means disposed at the distal ends of the leg members, said clip having audible and tactile indications of functioning and functional integrity, and said polypropylene having a crystallinity in excess of 45%.

6. A ligating clip in accordance with Claim 5 having a crystallinity in excess of 50%.

7. A method of producing an improved thermally formed, polyolefin, surgical device having both audible and tactile indications functioning comprising heating the formed device to a temperature of 90°C to 150°C for a period of time sufficient to increase the crystallinity of the polyolefin to at least 45%.

8. A method according to Claim 7 wherein the polyolefin is polypropylene.

9. A method according to Claim 7 or 8 wherein the device is an injection molded device.

10. A method according to any one of Claims 7 to 9 wherein the surgical device is a ligating clip.

11. A method according to Claim 10 wherein the ligating clip comprises two leg members connected at their proximal ends by a resilient hinge and containing locking means disposed at the distal ends of the leg members.

12. A method according to any one of Claims 7 to 11 wherein the device is heated to a temperature of from 100°C to 115°C.

13. A method according to any one of Claims 7 to 12 wherein the surgical device is heated for a period of time sufficient to increase the crystallinity to at least 50%.

14. A method according to any one of Claims 7 to 13 wherein the surgical device is heated for a period of time of at least twenty minutes.

15. A method according to any one of Claims 7 to 14 wherein the device is heated in the absence of any substantial oxygen or moisture.

16. A method according to any one of Claims 7 to 15 wherein the device is heated in an atmosphere of nitrogen.

## Patentansprüche

1. Thermisch geformter chirurgischer Gegenstand aus Polyolefin, der sowohl hörbare als auch fühlbare Funktionsanzeigen und funktionelle Integrität aufweist, wobei das Polyolefin eine Kristallinität von über 45% aufweist.

2. Spritzgegossener chirurgischer Gegenstand nach Anspruch 1, wobei das Polyolefin Polypropylen ist.

3. Chirurgischer Gegenstand nach Anspruch 1 oder Anspruch 2, wobei der chirurgische Gegenstand eine Verbindungsklammer ist.

4. Chirurgischer Gegenstand nach den Ansprüchen 1, 2 oder 3, wobei die Kristallinität größer als 50% ist.

5. Spritzgegossene Verbindungsklammer aus Polypropylen mit zwei Schenkelgliedern, die an ihren benachbarten Enden durch einen elastischen Scharnierbereich verbunden sind und die eine an den entfernten Enden der Schenkel angeordnete Verschließeinrichtung aufweisen, wobei die Klammer hörbare und fühlbare Funktionsanzeigen und funktionelle Integrität aufweist, und wobei das Polypropylen eine Kristallinität von über 45% aufweist.

6. Verbindungsklammer nach Anspruch 5 mit einer Kristallinität von über 50%.

7. Verfahren zur Herstellung eines verbesserten, thermisch geformten chirurgischen Gegenstands aus Polyolefin, der sowohl hörbare als auch fühlbare Funktionsanzeigen aufweist, durch Erhitzen des geformten Gegenstandes auf eine Temperatur von 90 bis 150°C für eine Zeit, die ausreicht, um die Kristallinität des Polyolefins auf mindestens 45% zu erhöhen.

8. Verfahren nach Anspruch 7, wobei das Polyolefin Polypropylen ist.

9. Verfahren nach Anspruch 7 oder 8, wobei der Gegenstand ein spritzgegossener Gegenstand ist.

10. Verfahren nach einem der Ansprüche 7 bis 9, wobei der chirurgische Gegenstand eine Verbindungsklammer ist.

11. Verfahren nach Anspruch 10, wobei die Verbindungsklammer zwei Schenkel umfaßt, die an ihren benachbarten Enden durch ein elastisches Scharnier verbunden sind und die eine an den entfernten Enden der Schenkel angeordnete Verschließeinrichtung enthalten.

12. Verfharen nach einem der Ansprüche 7 bis 11, wobei der Gegenstand bei einer Temperatur von 100 bis 115°C erwärmt wird.

13. Verfahren nach einem der Ansprüche 7 bis 12, wobei der chirurgische Gegenstand ausreichende Zeit zur Erhöhung der kristallinität auf mindestens 50% erwärmt wird.

14. Verfahren nach einem der Ansprüche 7 bis 13, wobei der chirurgische Gegenstand mindestens 20 Minuten lang erwärmt wird.

15. Verfahren nach einem der Ansprüche 7 bis 14, wobei der Gegenstand in Abwesenheit irgendeiner wesentlichen Menge Sauerstoff oder Feuchtigkeit erwärmt wird.

16. Verfahren nach einem der Ansprüche 7 bis 15, wobei der Gegenstand unter Stickstoff erwärmt wird.

## Revendications

1. Dispositif chirurgical en polyoléfine formé par voie thermique et possédant à la fois des indications auditives et tactiles de fonctionnement et un caractère unitaire de fonctionnement, ladite polyoléfine ayant une cristallinité supérieure à 45%.

2. Dispositif chirurgical moulé par injection selon la revendication 1, dans lequel la poly-oléfine est le polypropylène.

3. Dispositif chirurgical selon la revendication 1 ou 2, dans lequel le dispositif chirurgical est une pince de ligature.

4. Dispositif chirurgical selon la revendication 1, 2 ou 3, dans lequel la cristallinité est supérieure à 50%.

5. Pince de ligature en polypropylène moulée par injection comportant deux branches reliées à leurs extrémités proximales par une charnière flexible et contenant des moyens de verrouillage disposés aux extrémités distales des branches, ladite pince ayant des indications auditives et tactiles de fonctionnement et un caractère unitaire fonctionnel et ledit polypropylène présentant une cristallinité de plus de 45%.

6. Pince de ligature selon la revendication 5, ayant une cristallinité de plus de 50%.

7. Procédé de production d'un dispositif chirurgical en polyoléfine d'un type perfectionné et formé par voie thermique, ayant à la fois des indications auditives et tactiles de fonctionnement, qui consiste à chauffer le dispositif formé à une température de 90 à 150°C pendant une durée suffisante pour augmenter la cristallinité de la polyoléfine jusqu'à au moins 45%.

8. Procédé selon la revendication 7, dans lequel la polyoléfine est le polypropylène.

9. Procédé selon la revendication 7 ou 8, dans lequel le dispositif est une dispositif moulé par injection.

10. Procédé selon l'une quelconque des revendications 7 à 9, dans lequel le dispositif chirurgical est une pince de ligature.

11. Procédé selon la revendication 10, dans lequel la pince de ligature comprend deux branches reliées à leurs extrémités proximales par une charnière flexible et contenant des moyens de verrouillage disposés aux extrémités distales des branches.

12. Procédé selon l'une quelconque des revendications 7 à 11, dans lequel on chauffe le dispositif à une température de 100 à 115°C.

13. Procédé selon l'une quelconque des revendications 7 à 12, dans lequel on chauffe le dispositif chirurgical pendant une durée suffisante pour en augmenter la cristallinité jusqu'à 50% au moins.

14. Procédé selon l'une quelconque des revendications 7 à 13 dans lequel on chauffe le dispositif chirurgical pendant au moins 20 minutes.

15. Procédé selon l'une des revendications 7 à 14 dans lequel on chauffe le dispositif en l'absence d'une quantité notable d'oxygène ou d'humidité.

16. Procédé selon l'une des revendications 7 à 15, dans lequel on chauffe le dispositif dans une atmosphère d'azote.

0 072 232

Fig.1.

Fig.2.

Fig.3.

Fig.4.

Fig.5.

1